(19) Europäisches Patentamt
European Patent Office
Office européen des brevets

(11) Veröffentlichungsnummer: **0 062 614**
**B1**

# (12) EUROPÄISCHE PATENTSCHRIFT

(45) Veröffentlichungstag der Patentschrift: **19.06.85**

(51) Int. Cl.[4]: **C 09 B 57/04**, C 07 D 209/44, C 07 D 403/12

(21) Anmeldenummer: **82810146.9**

(22) Anmeldetag: **01.04.82**

(54) **Verfahren zur Herstellung von Bismethinisoindolinen.**

(30) Priorität: **06.04.81 CH 2328/81**

(43) Veröffentlichungstag der Anmeldung: **13.10.82 Patentblatt 82/41**

(45) Bekanntmachung des Hinweises auf die Patenterteilung: **19.06.85 Patentblatt 85/25**

(84) Benannte Vertragsstaaten: **CH DE FR GB IT LI**

(56) Entgegenhaltungen:
**EP-A-0 019 588**
**DE-A-1 914 271**
**DE-B-1 268 621**
**FR-A-2 412 589**
**GB-A-1 187 667**

(73) Patentinhaber: **CIBA-GEIGY AG, Postfach, CH-4002 Basel (CH)**

(72) Erfinder: **Rochat, Alain Claude, Dr., Bruderholzallee 25, CH-4059 Basel (CH)**
Erfinder: **Cassar, Luigi, Dr., Violaweg 81, CH-4303 Kaiseraugst (CH)**

**EP 0 062 614 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Bismethinisoindolinen aus Phthalonitrilen oder Diiminoisoindolinen und aktiven Methylenverbindungen.

Von Phthalonitrilen ausgehende Verfahren, in denen die Methylenkomponenten unter neutralen, aber bevorzugt sauren (DE-B 2 041 999) Bedingungen ankondensiert werden, sind bekannt. So wird in der EP-A 019 588, Beispiel 6, ein Verfahren zur Herstellung eines Bismethinisoindolins aus Phthalonitril beschrieben, in dem die beiden Methylenverbindungen in Gegenwart einer Säure ankondensiert werden. Die Ausbeute beträgt 67%, und das erhaltene Bismethinisoindolin muß vor seiner Verwendung gemahlen werden. Ferner ist aus J. Chem. Soc., 1940, S. 1078, Beispiele 2 und 3, die Umsetzung von Phthalonitril mit einer aktiven Methylenkomponente in Gegenwart einer Base bekannt. Nach diesem Verfahren wird aber nur das Monokondensationsprodukt erhalten. Zudem sind gemäß DE-B 1 268 621, Spalte 7, Versuchsbericht, die erhaltenen Produkte von ungenügender Reinheit, so daß von einem alkalischen Verfahren überhaupt abgeraten wird.

Aufgabe der Erfindung war es nun, ein vereinfachtes und verbessertes Verfahren zu finden, das in hoher Ausbeute reine und feinkristalline Bismethinisoindoline liefert. Die Lösung wurde darin gefunden, die Kondensation gerade in Gegenwart einer Base durchzuführen.

Die vorliegende Erfindung betrifft somit ein vereinfachtes und verbessertes Verfahren zur Herstellung von Bismethinisoindolinen der Formel

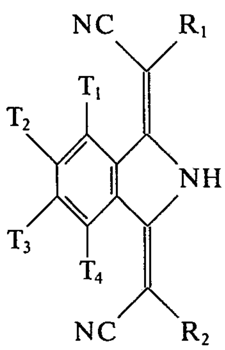
(I)

worin $T_1$ bis $T_4$ Wasserstoff oder Halogen bedeuten, oder einer oder zwei der Reste $T_1$ bis $T_4$ Alkyl, Alkoxy oder Phenoxy bedeuten, und $R_1$ und $R_2$ unabhängig voneinander Cyano, Alkyl- oder Arylcarbonyloxy, Carbamoyl, Alkylcarbamoyl oder Reste der Formeln II—IV sind,

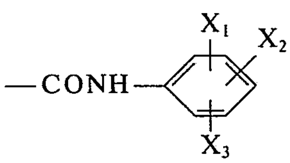
(II)
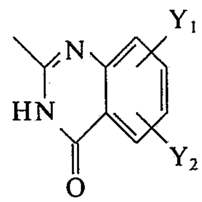
(III)
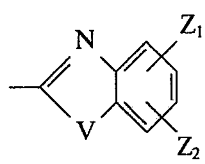
(IV)

worin $X_1$ eine nicht-löslichmachende Gruppe ist, und $X_2$ und $X_3$ Wasserstoff, Halogen, Alkyl, Alkoxy oder Alkoxycarbonyl sind oder zusammen eine —NR'—CO—NR''-Gruppe bilden, worin R' und R'' unabhängig voneinander Wasserstoff oder $C_1$—$C_4$-Alkyl sind, und $Y_1$ und $Y_2$ Wasserstoff, Halogen, Alkyl oder Alkoxy bedeuten oder zusammen einen ankondensierten Benzolring bilden, und V Sauerstoff, Schwefel, Imino oder Alkylimino mit 1 bis 4 C Atomen ist, und $Z_1$ und $Z_2$ die Bedeutung von $Y_1$ und $Y_2$ haben, dadurch gekennzeichnet, daß man die Kondensation von 1 Mol einer Verbindung der Formel V, VI oder VII oder der Salze von VI oder VII

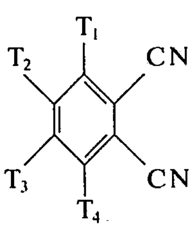
(V)
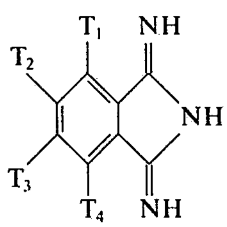
(VI)
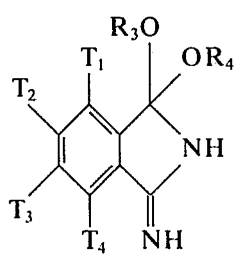
(VII)

worin $T_1$ bis $T_4$ die oben angegebene Bedeutung haben, und $R_3$ und $R_4$ gleich oder verschieden sind und $C_1$—$C_4$-Alkyl bedeuten oder zusammen die Gruppe $-CH_2-CH_2-$ bilden, mit 2 Mol einer Cyano-

methylenverbindung der Formel

$NC-CH_2-R_1$ (VIII) oder $NC-CH_2-R_2$ (IX)

worin $R_1$ und $R_2$ die oben angegebene Bedeutung haben, oder mit 1 Mol einer Cyanomethylenverbindung der Formel VIII und 1 Mol einer Cyanomethylenverbindung der Formel IX in einem polaren Lösungsmittel in Gegenwart einer starken Base durchführt, und das erhaltene Bismethinisoindolin durch Hydrolyse isoliert.

$T_1$ bis $T_4$ sind als Halogen insbesondere Chlor. Sofern $T_1$ bis $T_4$ Alkyl bedeuten, so handelt es sich insbesondere um solches mit 1 bis 6 C-Atomen, wie Äthyl, n-Propyl und vor allem Methyl. $T_1$ bis $T_4$ in der Bedeutung von Alkoxy haben insbesondere 1 bis 6 C-Atome, wie Äthoxy und vor allem Methoxy.

Alkyl in Alkylcarbamoyl und Alkylcarbonyloxy enthält insbesondere 1 bis 6 C-Atome, wie Äthyl, n-Propyl, Isopropyl, Hexyl und vor allem Methyl. Aryl in Acrylcarbonyloxy bedeutet vor allem Phenyl.

Unter $X_1$ als einer nicht-löslichmachenden Gruppe versteht man eine solche, die weder in Wasser noch in organischen Lösungsmitteln eine Lösung des Pigmentes bewirkt. Mögliche Gruppen sind beispielsweise Halogen, wie Fluor, Brom oder Chlor, Alkyl mit 1 bis 6 C-Atomen, wie Methyl, Äthyl oder Isopropyl, ferner Alkoxy mit 1 bis 6 C-Atomen, wie Methoxy oder Äthoxy, ferner Nitro, Trifluormethyl, Carbamoyl, Ureido, Sulfamoyl oder Cyano, ferner Alkoxycarbonyl, Alkanoyl, Alkylcarbamoyl, Alkylureido, Alkanoylamino, Alkylsulfonyl oder Alkylsulfamoyl, wobei die Alkylreste jeweils 1 bis 6 C-Atome enthalten und beispielsweise Methyl, Äthyl, Propyl, Butyl oder Hexyl bedeuten, ferner Aryl, Aryloxycarbonyl, Aroyl, Aroylamino, Arylsulfonyl, Arylcarbamoyl, Arylsulfamoyl, Arylureido oder Arylazo, wobei Aryl jeweils insbesondere Phenyl bedeutet und gegebenenfalls durch Methyl oder Chlor substituiert sein kann, und ferner eine im Phenylrest gegebenenfalls mit Chlor substituierte Phthalimidgruppe.

$X_2$ und $X_3$ bedeuten als Halogen vor allem Fluor, Brom oder Chlor, $X_2$ und $X_3$ haben als Alkyl oder Alkoxy insbesondere 1 bis 4 C-Atome, wie Methyl, Äthyl oder Butyl oder Methoxy, Äthoxy oder Butoxy, und als Alkoxycarbonyl 2 bis 5 C-Atome, wie vor allem Methoxycarbonyl oder Äthoxycarbonyl. In der $-NR'-CO-NR''$-Gruppe, die $X_2$ und $X_3$ zusammen bilden können, sind R' und R'' in der Bedeutung von $C_1-C_4$-Alkyl vor allem Methyl.

$Y_1$ und $Y_2$ als Halogen, Alkyl oder Alkoxy haben die gleiche Bedeutung wie $X_2$ und $X_3$.

V als Alkylimino enthält insbesondere 1 bis 4 C-Atome und bedeutet vor allem Methylimino.

$R_3$ und $R_4$ als Alkyl bedeuten vor allem Methyl.

Als Salze der Verbindungen der Formeln VI und VII kommen insbesondere jene von den im erfindungsgemäßen Verfahren eingesetzten starken Basen in Frage, wie sie unten beschrieben sind.

Vorzugsweise verwendet man zur erfindungsgemäßen Herstellung von Bismethinisoindolinen der Formel I als Ausgangsstoffe Verbindungen der Formel V, VI oder VII, insbesondere aber der Formel V, worin $T_1$ bis $T_4$ Wasserstoff und $R_3$ und $R_4$ Methyl bedeuten und Cyanomethylverbindungen der Formeln VIII bis IX, worin $R_1$ und $R_2$ Reste der Formeln II bis IV sind, wobei $X_1$ Wasserstoff, Fluor, Brom, Chlor, Carbamoyl, unsubstituiertes oder durch Chlor oder Methyl substituiertes Phenylcarbamoyl ist, $X_2$ und $X_3$ Wasserstoff, Fluor, Brom oder Chlor bedeuten oder zusammen eine Carbodiiminogruppe bilden, wobei ein Iminorest mit Methyl substituiert sein kann, und $Y_1$, $Y_2$, $Z_1$ und $Z_2$ Wasserstoff, Brom, Chlor, Methyl oder Methoxy sind, und V Imino bedeutet.

Als Ausgangsstoffe sind besonders Cyanomethylenverbindungen der Formeln VIII bis IX mit den Resten $R_1$ und $R_2$ der Formeln II und III, insbesondere der Formel II, bevorzugt, worin $X_1$ bis $X_3$ und $Y_1$ und $Y_2$ Wasserstoff oder Chlor bedeuten.

Vor allem verwendet man als Ausgangsstoffe neben einer Verbindung der Formel V, worin $T_1$ bis $T_4$ Wasserstoff bedeuten, bevorzugt eine Cyanomethylenverbindung der Formel X

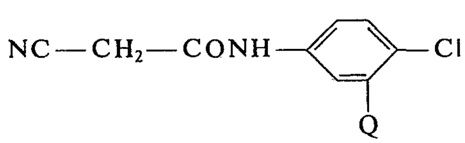

(X)

worin Q Wasserstoff oder Chlor bedeutet, oder eine Cyanomethylenverbindung der Formel X zusammen mit einer solchen der Formel XI

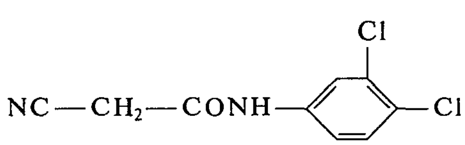

(XI)

Man führt die Kondensation in einem polaren Lösungsmittel durch, beispielsweise in einem aliphatischen Alkohol mit 1 bis 4 C-Atomen, wie Methanol, Äthanol, Isopropanol oder Butanol, ferner in einem Glykol, wie Äthylenglykol oder Diäthylenglykol, ferner in einem Glykoläther, wie Äthylenglykol-methyl-

äther, Äthylenglykol-äthyläther, Diäthylenglykol-monomethyläther oder Diäthylenglykol-monoäthyläther, oder ferner in einem dipolar-aprotischen Lösungsmittel, wie Acetonitril, Benzonitril, Dimethylformamid, N,N-Dimethylacetamid, Nitrobenzol, Dimethylsulfoxid oder N-Methylpyrrolidon. Es können auch Mischungen der genannten Lösungsmittel verwendet werden. Zweckmäßigerweise verwendet man 5—20 Gew.-Teile Lösungsmittel auf 1 Gew.-Teil der Reaktionsteilnehmer.

Unter bestimmten Bedingungen ist es auch möglich, die genannten Lösungsmittel zusammen mit Wasser zu verwenden. Bevorzugte polare Lösungsmittel sind aliphatische Alkohole, Glykole und Glykoläther. Besonders bevorzugt verwendet man als polares Lösungsmittel Methanol.

Das erfindungsgemäße Verfahren wird in Gegenwart einer starken Base durchgeführt, die insbesondere in einer Menge von 0,9 bis 1,1 Moläquivalenten bezüglich des Reaktanden der Formel V, VI oder VII eingesetzt werden kann. In bestimmten Fällen kann es genügen, nur katalytische Mengen Base zu verwenden. Geeignete starke Basen sind z. B. Alkalihydroxide, wie Natrium-, Kalium- oder Lithiumhydroxid, oder Erdalkalihydroxide, wie Calcium- oder Magnesiumhydroxid, oder Alkalicarbonate, wie Natrium-, Kalium- oder Lithiumcarbonat, oder Alkalialkoholate mit 1 bis 6 C-Atomen, wie Natrium-, Kalium- oder Lithiummethylat, -äthylat, -propylat oder -tert.-butylat, oder tert.-Alkylamine mit insgesamt 3 bis 18 C-Atomen, wie Triäthylamin, oder Alkaliamide, wie Lithium- oder Natriumamid, oder Tetraalkylammoniumhydroxide mit 4 bis 24 C-Atomen, wie Tetramethylammoniumhydroxid.

Bevorzugte Basen sind die Alkalihydroxide, Alkalicarbonate und Alkalialkoholate.

Die genannten Basen können zusammen mit einem Phasentransferkatalysator eingesetzt werden. Dies ist vor allem dann von Vorteil, wenn die Löslichkeit einer bestimmten Base in einem bestimmten Lösungsmittel gering ist. Insbesondere Alkalicarbonate in aliphatischen Alkoholen oder Glykolen werden bevorzugt zusammen mit einem Phasentransferkatalysator verwendet. Die Phasentransferkatalysatoren können in einer Menge von 0,001 bis 50 Mol-%, vorzugsweise 0,01 bis 0,3 Mol-%, bezogen auf die Reaktanden der Formel V, VI oder VII eingesetzt werden. Für das erfindungsgemäße Verfahren eignen sich die üblichen, in der Literatur beschriebenen Phasentransferkatalysatoren, wie sie z. B. in CHEMTECH, Februar 1980, S. 111, Tabelle 1, aufgeführt sind, nämlich beispielsweise quaternäre Salze, cyclische Polyäther, offenkettige Polyäther, N-Alkylphosphoramide oder mit Methylen überbrückte Phosphor- oder Schwefeloxide. Bevorzugt verwendet man als Phasentransferkatalysatoren quaternäre Salze, wie quaternäre Ammonium- oder Phosphoniumsalze, wobei es sich insbesondere um die Halogenide handelt.

Beispiele für quaternäre Ammonium- bzw. Phosphoniumsalze sind: Tetrabutylammoniumhydrogensulfat, Tetrabutyl-, Tetrahexyl-, Trioctylmethyl-, Trioctyläthyl-, Hexyltriäthyl-, Octyltriäthyl-, Decyltriäthyl-, Dodecyltriäthyl-, Hexadecyltrimethyl-, Benzyltriäthyl-, Tricaprylmethyl- und Triphenylmethylammonium- bzw. -phosphonium, -chlorid, -bromid- und -jodid.

Das erfindungsgemäße Verfahren kann insbesondere bei Temperaturen von 0° bis 180°C, vorzugsweise 50 bis 110°C durchgeführt werden.

Zur Hydrolyse des Kondensationsproduktes kann man Wasser, vorzugsweise aber eine Säure verwenden. Als Säuren kommen z. B. aliphatische und aromatische Carbon- oder Sulfonsäuren in Betracht, wie beispielsweise Ameisensäure, Essigsäure, Propionsäure, Oxalsäure, Benzoesäure oder Benzolsulfonsäure.

Weiterhin kommen als Säuren auch Mineralsäuren, wie Salzsäure, Schwefelsäure oder Phosphorsäure in Betracht.

Bei der Hydrolyse fällt das Bismethinisoindolin aus und kann durch Abfiltrieren isoliert werden.

Die Kondensation der Verbindung der Formel V, VI oder VII mit den Cyanomethylenverbindungen erfolgt vorzugsweise über alle Reaktionsstufen im gleichen Reaktionsmedium. Kondensation im gleichen Reaktionsmedium bedeutet, daß die Ausgangsstoffe direkt zum Endstoff umgesetzt werden, ohne Kondensationszwischenprodukte zu isolieren.

Eine weitere Ausführungsform des Verfahrens besteht darin, unabhängig voneinander hergestellte, verschiedene Reaktionslösungen vor der Hydrolyse zu mischen und zusammen zu hydrolisieren. Es ist möglich, unabhängig voneinander hergestellte, verschiedene Kondensationsprodukte vor der Hydrolyse als Salze zu isolieren, zusammenzumischen und dann zu hydrolysieren. Man erhält dabei ein Gemisch von Bismethinisoindolinen.

Die im erfindungsgemäßen Verfahren eingesetzten Ausgangsstoffe sind bekannte Verbindungen und können nach bekannten Verfahren hergestellt werden.

Nach dem erfindungsgemäßen Verfahren fallen die Bismethinisoindoline bzw. Bismethinisoindolingemische in der Regel in einer direkt verwendbaren Pigmentform an. Dabei lassen sich mehr transparente oder mehr deckende Pigmentformen erzielen.

Um eine transparente Pigmentform zu erhalten, wird die Hydrolyse bevorzugt bei tiefen Temperaturen (unter 80°C) durchgeführt.

Wird eine deckende Pigmentform gewünscht, so erweist sich eine Hydrolyse bei höherer Temperatur (über 80°C), gegebenenfalls unter Druck, als zweckmäßig. Man kann auch das Pigment nach der Hydrolyse mit einem zusätzlichen Lösungsmittel versetzen und erwärmen, oder zuerst isolieren und nachträglich in Wasser oder in einem organischen Lösungsmittel erwärmen, gegebenenfalls unter Druck, um die deckende Form zu erlangen. Vorzugsweise verwendet man solche organischen Lösungsmittel, die über 80°C sieden. Als besonders geeignet erweisen sich durch Halogenatome, Alkyl- oder

Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Äther, wie Äthylenglykolmonomethyl- oder -monoäthyläther, Amide, wie Dimethylformamid oder N-Methylpyrrolidon, sowie Dimethylsulfoxyd oder Sulfolan. Man kann die Nachbehandlung auch in Wasser in Gegenwart von organischen Lösungsmitteln und/oder mit Zusatz von oberflächenaktiven Substanzen durchführen. Solche Lösungsmittel-Nachbehandlungen haben öfters eine gute Wirkung auf das Glanzverhalten der Pigmente in Lackfärbungen.

Die nach dem erfindungsgemäßen Verfahren erhaltenen Stoffe stellen wertvolle Pigmente mit hoher Farbstärke, Farbtonreinheit, Licht-, Wetter- und Überlackierechtheit sowie hohem Glanz dar, die in der Regel ohne zusätzliche Maßnahmen direkt zum Pigmentieren von hochmolekularen organischen Materialien, insbesondere von Lacken, verwendet werden können.

Die folgenden Beispiele erläutern die Erfindung.

## Beispiel 1

12,82 g Phthalonitril und 19,45 g Cyanessigsäure-4-chloranilid werden in 200 ml Äthylenglykolmonoäthyläther während 30 Minuten bei 20—25°C gerührt. Die Suspension versetzt man innert 25 Minuten mit 18 g einer 30,8gew.-%igen Lösung von Natriummethylat in Methanol, dann rührt man während 1 Stunde bei 30°C und während 30 Minuten bei 40°C. Nach dem Abkühlen auf 30°C versetzt man das Reaktionsgemisch mit 21,4 g Cyanessigsäure-4-chloranilid. Das Gemisch wird innert 2 Stunden auf 110°C erwärmt und während 2½ Stunden bei dieser Temperatur gerührt. Nach dem Abkühlen auf 60°C gibt man die Reaktionslösung innert 15 Minuten in ein Gemisch von 8,4 g Essigsäure und 200 ml Methanol und kocht die Suspension während 30 Minuten bei Rückflußtemperatur. Nach dem Abkühlen auf 50°C wird das erhaltene Pigment abfiltriert, mit 550 ml warmem Methanol und mit 300 ml warmem Wasser gewaschen. Nach dem Trocknen im Vakuum bei 70—80°C erhält man 47,97 g (95,9% der Theorie, bezogen auf Phthalonitril) reines orange-rotes Pigment der Formel XII

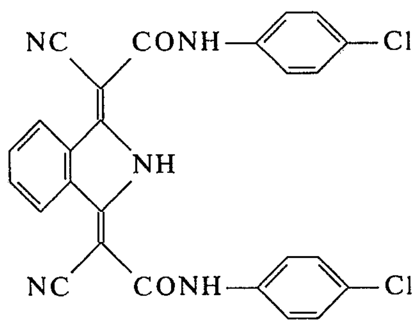

(XII)

Mikroanalyse:

| | |
|---|---|
| berechnet: | C 62,4% H 3,0% N 14,0% O 6,4% Cl 14,2% |
| gefunden: | C 62,3% H 3,3% N 13,9% O 6,8% Cl 14,0% |

Das Pigment hat eine spezifische Oberfläche von ca. 56 $m^2/g$ und kann als solches in Lacken weiterverarbeitet werden, wobei transparente und farbstarke Färbungen erhalten werden. Durch einstündiges Erwärmen des Pigmentes in o-Dichlorbenzol bei 100°C wird ein deckenderes Pigment mit einer spezifischen Oberfläche von ca. 20 $m^2/g$ erhalten, das sehr brillante, wetterechte und glänzende Färbungen ergibt.

## Beispiel 2

6,4 g Phthalonitril und 21,4 g Cyanessigsäure-4-chloranilid in 130 ml Methanol werden innert 20 Minuten bei 30°C mit einer Lösung von 2,1 g Lithiumhydroxid-Monohydrat in 20 ml Methanol versetzt. Das Reaktionsgemisch wird zuerst während 15½ Stunden bei 60—65°C, dann während 1 Stunde bei 0°C gerührt. Man filtriert das Reaktionsgemisch bei 0°C und wäscht den Filterkuchen mit 50 ml eiskaltem Methanol. Der Filterkuchen wird in 130 ml Methanol aufgeschlämmt, auf Rückflußtemperatur erwärmt, mit einer Mischung von 4,4 ml Essigsäure und 4,4 ml Methanol versetzt und während 30 Minuten bei Rückflußtemperatur gekocht. Nach dem Abkühlen auf 50°C wird das erhaltene Pigment abfiltriert, mit 450 ml warmem Methanol und 150 ml warmem Wasser gewaschen. Nach dem Trocknen im Vakuum bei 70—80°C erhält man 23,1 g (92,4% der Theorie, bezogen auf Phthalonitril) reines orange-rotes Pigment der Formel XII.

Mikroanalyse:

| | |
|---|---|
| berechnet: | C 62,4% H 3,0% N 14,0% O 6,4% Cl 14,2% |
| gefunden: | C 62,3% H 3,1% N 14,1% O 6,4% Cl 13,9% |

Beispiel 3

Ein Gemisch von 12,8 g Phthalonitril, 19,45 g Cyanessigsäure-4-chloranilid, 27,5 g Cyanessigsäure-3,4-dichloranilid, 13,82 g gemahlenes Kaliumcarbonat und 0,8 g Tricaprylmethylammoniumchlorid in 300 ml Äthylenglykol wird innert 6 Stunden auf 100°C erwärmt. Die Suspension wird 1 Stunde bei 100°C gerührt, dann auf 70°C abgekühlt und langsam innert 10 Minuten zu einer 40°C warmen Mischung von 8,4 ml Essigsäure und 200 ml Methanol gegeben. Man kocht 30 Minuten bei Rückflußtemperatur, filtriert das erhaltene Pigment ab und wäscht es mit 600 ml warmem Methanol und mit 250 ml warmem Wasser. Nach dem Trocknen erhält man 52,1 g (97,5% der Theorie, bezogen auf Phthalonitril) eines direkt als Pigment verwendbaren orangen Gemisches, bestehend hauptsächlich aus dem Pigment der Formel XIII

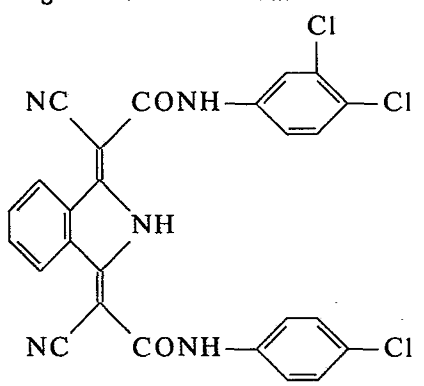

(XIII)

Mikroanalyse:

| | |
|---|---|
| berechnet: | C 58,4% H 2,6% N 13,1% O 6,0% Cl 19,9% |
| gefunden: | C 57,8% H 2,9% N 12,9% O 6,1% Cl 20,4% |

Beispiel 4

0,98 g Phthalonitril in 15 ml Methanol werden mit 1,32 g einer 30,8gew.-%igen Lösung von Natriummethylat in Methanol versetzt, wobei sich das 1,1-Dimethoxy-3-imino-isoindolin in Form des Natriumsalzes mit hoher Ausbeute bildet. (Siehe Angew. Chem., 68, 134—135 und 138—140 (1956).) Nach 30 Minuten Rühren bei 20—25°C gibt man 3,63 g 5-Cyanoacetamino-1-methyl-benzimidazolon-2 zu. Die Suspension wird zuerst während 90 Minuten bei 30°C gerührt, dann während 90 Minuten bei Rückflußtemperatur gekocht. Man fügt 20 ml Äthylenglykol-monoäthyläther zu und erwärmt das Gemisch auf 85°C, wobei das Methanol abdestilliert wird. Man rührt noch während 30 Minuten bei 85°C und kühlt dann auf 40°C ab. Die Suspension wird mit 25 ml Methanol und 3,9 ml Essigsäure versetzt und während 30 Minuten bei Rückflußtemperatur gekocht. Nach dem Abkühlen auf 50°C wird das erhaltene Pigment abfiltriert und mit 200 ml warmem Methanol und mit 100 ml warmem Wasser gewaschen. Nach dem Trocknen erhält man 3,41 g (79,7% der Theorie, bezogen auf Phthalonitril) reines braunes Pigment der Formel XIV

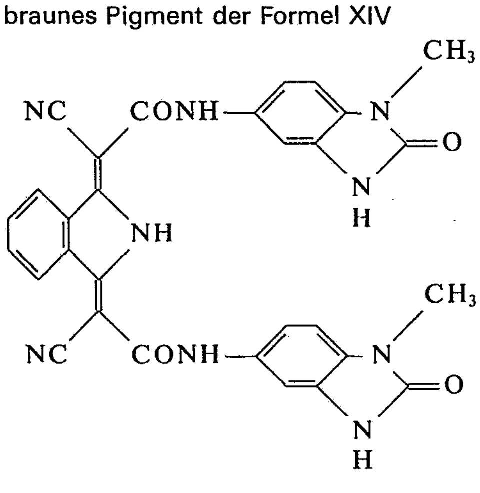

(XIV)

Mikroanalyse:

| | |
|---|---|
| berechnet: | C 61,1% H 3,9% N 21,4% O 13,6% |
| gefunden: | C 60,6% H 4,0% N 21,9% O 13,2% |

Beispiel 5

7,86 g 82%iges Benzimidazol-2-yl-acetonitril werden in 30 ml wasserfreiem Dimethylsulfoxid bei 20—25°C gelöst und anschließend innert 7 Minuten mit 5,61 g Kalium-tert.-butylat versetzt. Man rührt während 12 Minuten bei 20—25°C und gibt 6,4 g Phthalonitril zu. Die Reaktionslösung wird stufenweise auf 80°C erwärmt und 70 Minuten bei dieser Temperatur gerührt. Nach dem Abkühlen auf 50°C versetzt man die Reaktionslösung mit 7,86 g 82%igem Benzimidazol-2-yl-acetonitril, erwärmt auf 120°C und rührt bei dieser Temperatur während 155 Minuten. Nach dem Abkühlen auf 75°C gibt man zur Reaktionslösung 6 ml Essigsäure und 120 ml Methanol und kocht während 30 Minuten bei Rückflußtemperatur. Nach dem Abkühlen auf 40°C wird das erhaltene Pigment filtriert, dann mit 100 ml Methanol und einem Gemisch von 50 ml Methanol und 50 ml Wasser und schließlich mit 50 ml Wasser gewaschen. Nach dem Trocknen im Vakuum bei 70°C erhält man 15,95 g (91,5% der Theorie, bezogen auf 100%iges Benzimidazol-2-yl-acetonitril) reines blaustichigrotes Pigment der Formel XV

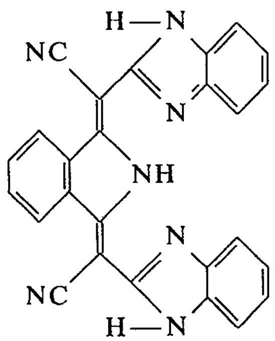

(XV)

Mikroanalyse:

| | |
|---|---|
| berechnet: | C 73,4% H 3,6% N 23,1% |
| gefunden: | C 72,7% H 3,5% N 22,6% |

Beispiel 6

Zu 3,2 g Phthalonitril und 10,2 g 2-Cyanmethylchinazolon in 60 ml Äthylenglykol gibt man 4,38 g einer 30,8gew.-%igen Lösung von Natriummethylat in Methanol und rührt die Suspension während 30 Minuten bei 20—25°C. Man erwärmt auf 60°C, rührt während 2 Stunden bei dieser Temperatur, erwärmt weiter auf 90°C und hält diese Temperatur während weiteren 2 Stunden und rührt schließlich während 3 Stunden bei 110°C. Nach dem Abkühlen auf 80°C wird die Suspension in eine 40°C warme Mischung von 1,9 ml Essigsäure und 100 ml Äthylenglykol-monoäthyläther gegeben. Man rührt während 30 Minuten bei 40°C und während 1 Stunde bei 65°C. Das erhaltene Pigment wird warm abfiltriert und mit 250 ml Äthylenglykol-monoäthyläther, 400 ml Methanol und 100 ml Wasser gewaschen. Nach dem Trocknen erhält man 9,4 g (78,1% der Theorie, bezogen auf Phthalonitril) reines orange-braunes Pigment der Formel XVI

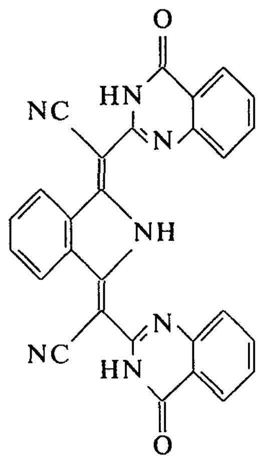

(XVI)

Mikroanalyse:

| | | | | |
|---|---|---|---|---|
| berechnet: | C 69,9% | H 3,1% | N 20,4% | O 6,6% |
| gefunden: | C 68,3% | H 3,5% | N 21,0% | O 7,1% |

Beispiel 7

12,8 g Phthalonitril in 200 ml Äthylenglykol werden mit 17,54 g einer 30,8gew.-%igen Lösung von Natriummethylat in Methanol versetzt. Nach 30 Minuten Rühren bei 20—25°C gibt man 27,5 g Cyanessigsäure-3,4-dichloranilid hinzu und rührt das Gemisch während 2½ Stunden bei 28—30°C. Anschließend werden 19,45 g Cyanessigsäure-4-chloranilid dazugegeben, und das Gemisch wird innert 30 Minuten auf 65°C erwärmt. Nach 2 Stunden bei 65°C erwärmt man die Suspension innert 30 Minuten auf 100°C und hält diese Temperatur während 2 Stunden. Die Suspension wird auf 60°C abgekühlt und innert ca. 5 Minuten zu einer 40°C warmen Mischung von 7,2 ml Essigsäure und 300 ml Methanol gegeben. Nach 30 Minuten Rühren erwärmt man 30 Minuten bei Rückflußtemperatur, kühlt auf ca. 50°C ab und filtriert das Pigment ab. Man wäscht es mit 400 ml warmem Methanol und mit 300 ml warmem Wasser. Nach dem Trocknen erhält man 52,9 g (99,0% der Theorie, bezogen auf Phthalonitril) eines orangen Gemisches, bestehend hauptsächlich aus dem Pigment der Formel XIII. Das Pigment weist eine spezifische Oberfläche von ca. 53 $m^2/g$ auf und kann als solches in Lacken weiterverarbeitet werden, wobei transparente und farbstarke Färbungen erhalten werden.

Mikroanalyse:

| | | | | |
|---|---|---|---|---|
| berechnet: | C 58,4% | H 2,6% | N 13,1% | Cl 19,9% |
| gefunden: | C 57,6% | H 2,5% | N 13,0% | Cl 20,1% |

Durch zweistündiges Erwärmen bei 130°C in o-Dichlorbenzol wird ein wesentlich deckenderes Pigment mit einer spezifischen Oberfläche von etwa 20 $m^2/g$ erhalten.

Beispiel 8

Ein Gemisch von 6,4 g Phthalonitril, 10,1 g Cyanacetamid, 2,0 g Ätznatron und 100 ml Äthanol absolut wird während 19 Stunden bei Rückflußtemperatur (ca. 76°C) gekocht. Dann läßt man die Suspension auf 50°C abkühlen, versetzt es mit 6 ml Essigsäure und kocht es wieder kurz bei Rückflußtemperatur. Das Pigment wird anschließend filtriert, mit 150 ml warmem Äthanol und 100 ml warmem Wasser gewaschen und im Vakuum bei 80°C getrocknet. Man erhält 13,6 g (97,5% der Theorie, bezogen auf Phthalonitril) reines grünstichiggelbes Pigment der Formel XVII.

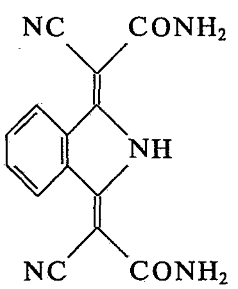

(XVII)

Mikroanalyse:

| | | | |
|---|---|---|---|
| berechnet: | C 60,22% | H 3,25% | N 25,08% |
| gefunden: | C 60,0 % | H 3,50% | N 24,70% |

Beispiel 9

Ein Gemisch von 6,4 g Phthalonitril, 21,4 g Cyanessigsäure-4-chloranilid, 7,6 g gemahlenem Kaliumcarbonat und 1,4 g Polyoxyäthylenlauryläther (Brij 35®) in 130 ml Methanol wird in 30 Minuten auf Rückflußtemperatur erwärmt (ca. 65°C) und während 10 Stunden bei dieser Temperatur gekocht. Die erhaltene Pigmentsuspension wird auf 50°C abgekühlt und innert 10 Minuten zu einer 40°C warmen Mischung von 7,3 ml Essigsäure und 125 ml Methanol gegeben. Man kocht 30 Minuten bei Rückflußtemperatur, kühlt wieder auf ca. 50°C ab und filtriert das Pigment. Man wäscht es mit 350 ml warmem Methanol, dann mit 200 ml warmem Wasser und trocknet es im Vakuum bei 100°C. Die Ausbeute beträgt 24,38 g reines orange-rotes Pigment der Formel XII (97,5% der Theorie, bezogen auf Phthalonitril). Das Produkt ergibt transparente Lackfärbungen.

Mikroanalyse:

| | |
|---|---|
| berechnet: | C 62,4% H 3,0% N 14,0% O 6,4% Cl 14,2% |
| gefunden: | C 62,0% H 3,2% N 13,8% O 6,7% Cl 14,0% |

Wenn man die Pigmentsuspension nach der Zugabe in Essigsäure und Methanol 2 Stunden bei 95°C unter leichtem Druck (1,7 bar) erwärmt und anschließend wie oben aufarbeitet, erhält man 23,5 g reines Pigment der Formel XII entsprechend einer Ausbeute von 94% der Theorie, bezogen auf Phthalonitril. Das Produkt ergibt deckende Lackfärbungen. Beide Pigmentformen (transparent und deckend) sind als solche bei der Lack-Herstellung direkt einsetzbar.

Beispiel 10

Ein Gemisch von 6,4 g Phthalonitril, 9,73 g Cyanessigsäure-4-chloranilid, 13,75 g Cyanessigsäure-3,4-dichloranilid, 7,6 g gemahlenes Kaliumcarbonat und 1,5 g Polyoxyäthylenlauryläther (Brij 35®) in 160 ml Methanol wird innert 30 Minuten auf Rückflußtemperatur erwärmt und während 12 Stunden bei Rückflußtemperatur gekocht. Die erhaltene Pigment-Suspension wird auf 50°C abgekühlt und innert 10 Minuten zu einer 40°C warmen Mischung von 7,3 ml Essigsäure und 125 ml Methanol gegeben. Man kocht 30 Minuten bei Rückflußtemperatur, kühlt wieder auf ca. 50°C ab und filtriert das Pigment. Man wäscht es mit 400 ml warmem Methanol, dann mit 150 ml warmem Wasser und trocknet es im Vakuum bei 50°C. Die Ausbeute beträgt 26,7 g (99,9% der Theorie, bezogen auf Phthalonitril) eines orangen Gemisches, bestehend hauptsächlich aus dem Pigment der Formel XIII. Das Gemisch ist direkt zur Lack-Herstellung einsetzbar und ergibt sehr farbstarke und transparente Färbungen.

Mikroanalyse:

| | |
|---|---|
| berechnet: | C 58,4% H 2,6% N 13,1% O 6,0% Cl 19,9% |
| gefunden: | C 57,7% H 2,8% N 13,0% O 6,3% Cl 20,1% |

Beispiel 11

12,82 g Phthalonitril, 39,16 g Cyanessigsäure-3-fluoranilid und 15,18 g Kaliumcarbonat werden in 500 ml Methanol auf Rückflußtemperatur erwärmt. Nach 10 Stunden bei dieser Temperatur fügt man 20 ml Essigsäure zu und filtriert das rotstichiggelbe Pigment heiß ab. Man wäscht es mit Methanol und Wasser. Nach dem Trocknen erhält man 42,3 g Pigment der Formel XVIII

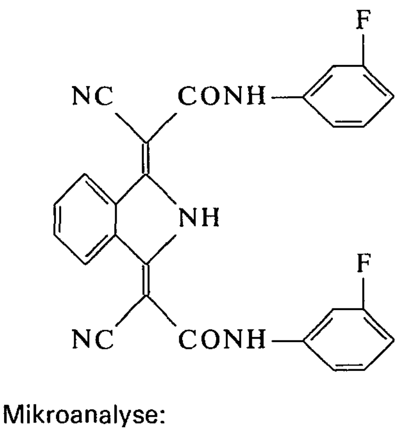

(XVIII)

Mikroanalyse:

| | |
|---|---|
| berechnet: | C 66,81% H 3,23% N 14,98% F 8,13% |
| gefunden: | C 66,6 % H 3,3 % N 15,20% F 7,9 % |

Das Pigment kann als solches in Lacken weiterverarbeitet werden, wobei farbstarke, rotstichiggelbe Färbungen erhalten werden. Durch einstündiges Erwärmen in o-Dichlorbenzol erhält man eine besonders deckende Pigmentform. Vorteilhaft wird das Pigment durch Mahlen oder Kneten in eine feindisperse Form übergeführt. Nach der Einarbeitung in Lacke erhält man farbstarke, deckende Färbungen mit hohem Glanz und hohen Echtheiten, insbesondere mit hervorragender Licht- und Wetterechtheit.

Beispiel 12

Ein Gemisch von 9,68 g 75gew.-%igem Phthalogenbrillantblau IF 3G® (ein 1,3-Diiminoisoindolin von Bayer), 21,4 g Cyanessigsäure-4-chloranilid, 7,6 g gemahlenem Kaliumcarbonat und 1,4 g Polyoxyäthylenlauryläther (Brij 35® von Atlas Powder) in 180 ml Methanol wird innert 30 Minuten auf Rückflußtemperatur erwärmt und während 18 Stunden bei dieser Temperatur gehalten. Man kühlt die Suspension auf 50°C ab und versetzt sie innert 10 Minuten mit einem Gemisch von 9,2 ml Eisessig und 25 ml Methanol. Man kocht 30 Minuten bei Rückflußtemperatur, kühlt auf 50°C ab und filtriert. Der Filterrückstand wird mit 200 ml warmem Methanol und mit 100 ml warmem Wasser gewaschen. Nach dem Trocknen im Vakuum bei 80°C erhält man 23,75 g (95% der Theorie, bezogen auf Diiminoisoindolin) reines Pigment der Formel XII.

Mikroanalyse:

| | | | | |
|---|---|---|---|---|
| berechnet: | C 62,4% | H 3,02% | N 14,0% | Cl 14,17% |
| gefunden: | C 62,2% | H 3,2 % | N 14,0% | Cl 14,0 % |

**Patentansprüche**

1. Verfahren zur Herstellung von Bismethinisoindolinen der Formel

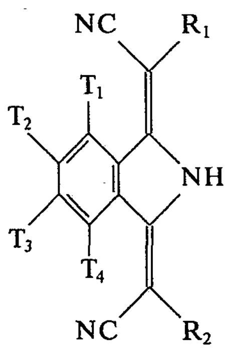

(I)

worin $T_1$ bis $T_4$ Wasserstoff oder Halogen bedeuten, oder einer oder zwei der Reste $T_1$ bis $T_4$ Alkyl, Alkoxy oder Phenoxy bedeuten, und $R_1$ und $R_2$ unabhängig voneinander Cyano, Alkyl- oder Arylcarbonyloxy, Carbamoyl, Alkylcarbamoyl oder Reste der Formeln II bis IV sind,

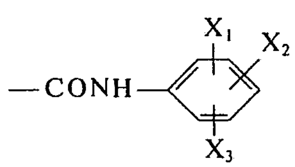 (II) 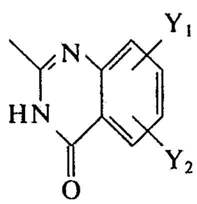 (III) 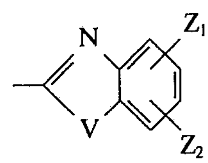 (IV)

worin $X_1$ eine nicht-löslichmachende Gruppe ist, und $X_2$ und $X_3$ Wasserstoff, Halogen, Alkyl, Alkoxy oder Alkoxycarbonyl sind oder zusammen eine –NR'–CO–NR''-Gruppe bilden, worin R' und R'' unabhängig voneinander Wasserstoff oder $C_1$–$C_4$-Alkyl sind, und $Y_1$ und $Y_2$ Wasserstoff, Halogen, Alkyl oder Alkoxy bedeuten oder zusammen einen ankondensierten Benzolring bilden, und V Sauerstoff, Schwefel, Imino oder Alkylimino mit 1 bis 4 C-Atomen ist, und $Z_1$ und $Z_2$ die Bedeutung von $Y_1$ und $Y_2$ haben, dadurch gekennzeichnet, daß man die Kondensation von 1 Mol einer Verbindung der Formel V, VI oder VII oder der Salze von VI oder VII

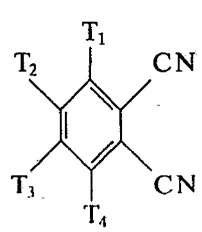 (V) 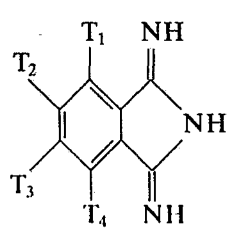 (VI) 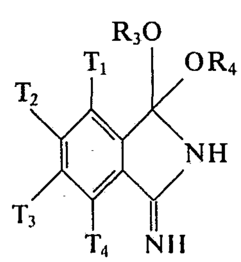 (VII)

worin $T_1$ bis $T_4$ die oben angegebene Bedeutung haben, und $R_3$ und $R_4$ gleich oder verschieden sind und $C_1-C_4$-Alkyl bedeuten oder zusammen die Gruppe $-CH_2-CH_2-$ bilden, mit 2 Mol einer Cyanomethylenverbindung der Formel

$$NC-CH_2-R_1 \quad (VIII) \qquad \text{oder} \qquad NC-CH_2-R_2 \quad (IX)$$

oder mit 1 Mol einer Cyanomethylenverbindung der Formel VIII und 1 Mol einer Cyanomethylenverbindung der Formel IX, wobei $R_1$ und $R_2$ die oben angegebene Bedeutung haben, in einem polaren Lösungsmittel in Gegenwart einer starken Base durchführt, und das erhaltene Bismethinisoindolin durch Hydrolyse aus dem Reaktionsgemisch isoliert.

2. Verfahren nach Anspruch 1, dadurch gekennzeichnet, daß man als Ausgangsstoff eine Verbindung der Formel V, VI oder VII verwendet, worin $T_1$ bis $T_4$ Wasserstoff und $R_3$ und $R_4$ Methyl bedeuten.

3. Verfahren nach Anspruch 1, dadurch gekennzeichnet, daß man als Ausgangsstoff eine Verbindung der Formel V verwendet, worin $T_1$ bis $T_4$ Wasserstoff bedeuten.

4. Verfahren nach Anspruch 1, dadurch gekennzeichnet, daß man als Ausgangsstoffe Cyanomethylenverbindungen der Formeln VIII bis IX mit den Resten $R_1$ und $R_2$ der Formeln II bis IV verwendet, worin $X_1$ Wasserstoff, Brom, Chlor, Carbamoyl oder unsubstituiertes oder durch Chlor oder Methyl substituiertes Phenylcarbamoyl ist, $X_2$ und $X_3$ Wasserstoff, Fluor, Brom oder Chlor bedeuten oder zusammen eine Carbodiiminogruppe bilden, wobei ein Iminorest mit Methyl substituiert sein kann, und $Y_1$, $Y_2$, $Z_1$ und $Z_2$ Wasserstoff, Brom, Chlor, Methyl oder Methoxy sind, und V Imino bedeutet.

5. Verfahren nach Anspruch 1, dadurch gekennzeichnet, daß man als Ausgangsstoffe Cyanomethylenverbindungen der Formeln VIII bis IX mit den Resten $R_1$ und $R_2$ der Formel II verwendet, worin $X_1$ bis $X_3$ Wasserstoff oder Chlor bedeuten.

6. Verfahren nach Anspruch 1, dadurch gekennzeichnet, daß man als Ausgangsstoffe unsubstituiertes Phthalonitril und Cyanomethylenverbindungen der Formeln

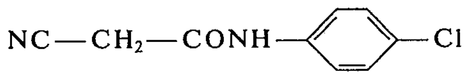

und

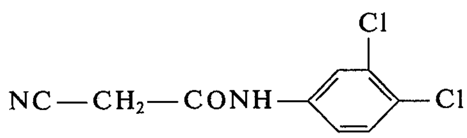

verwendet.

7. Verfahren nach Anspruch 1, dadurch gekennzeichnet, daß man als polares Lösungsmittel einen Alkohol verwendet.

8. Verfahren nach Anspruch 1, dadurch gekennzeichnet, daß man als starke Base ein Alkalihydroxid, Alkalicarbonat oder Alkalialkoholat verwendet.

9. Verfahren nach Anspruch 1, dadurch gekennzeichnet, daß man die Base zusammen mit einem Phasentransferkatalysator verwendet.

10. Verfahren nach Anspruch 1, dadurch gekennzeichnet, daß man die Kondensation über alle Reaktionsstufen im gleichen Reaktionsmedium durchführt.

11. Verfahren nach Anspruch 1, dadurch gekennzeichnet, daß man zur Hydrolyse eine organische Säure verwendet.

**Claims**

1. A process for the preparation of a bismethine isoindoline of the formula

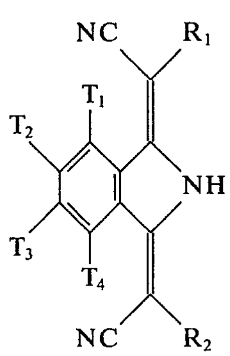

(I)

wherein $T_1$ to $T_4$ are hydrogen or halogen, or one or two of the radicals $T_1$ to $T_4$ are alkyl, alkoxy or phenoxy, and $R_1$ and $R_2$ are each independently cyano, alkyl- or arylcarbonyloxy, carbamoyl, alkylcarbamoyl, or radicals of the formulae II to IV

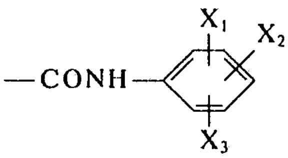 (II) 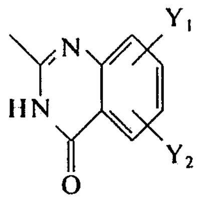 (III) 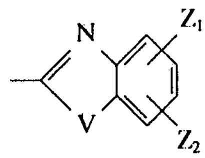 (IV)

wherein $X_1$ is a non-solubilising group, and $X_2$ and $X_3$ are hydrogen, halogen, alkyl, alkoxy or alkoxycarbonyl, or together form an —NR′—CO—NR″-group, in which R′ and R″ are each independently hydrogen or $C_1$—$C_4$-alkyl, and $Y_1$ and $Y_2$ are hydrogen, halogen, alkyl or alkoxy, or together form a fused benzene ring, V is oxygen, sulfur, imino or alkylimino having 1 to 4 C atoms, and $Z_1$ and $Z_2$ have the same meanings as $Y_1$ and $Y_2$, in which process the condensation of 1 mole of a compound of the formula V, VI or VII or of the salts of VI or VII

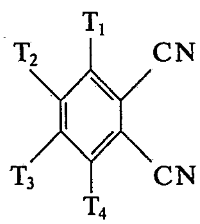 (V) 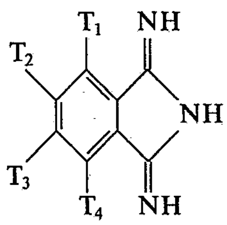 (VI) 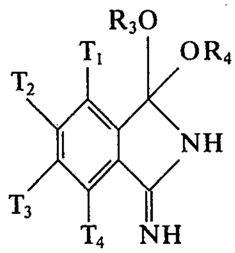 (VII)

wherein $T_1$ to $T_4$ are as defined above, and $R_3$ and $R_4$ are identical or different and are $C_1$—$C_4$-alkyl, or together form the $-CH_2-CH_2$-group, with 2 moles of a cyanomethylene compound of the formula

$NC-CH_2-R_1$ (VIII) or $NC-CH_2-R_2$ (IX)

or with 1 mole of a cyanomethylene compound of the formula VIII and 1 mole of a cyanomethylene compound of the formula IX, wherein $R_1$ and $R_2$ are as defined above, is performed in a polar solvent in the presence of a strong base, and the bismethine isoindoline obtained is isolated from the reaction mixture by hydrolysis.

2. A process according to claim 1, wherein the starting material is a compound of the formula V, VI or VII in which $T_1$ to $T_4$ are hydrogen, and $R_3$ and $R_4$ are methyl.

3. A process according to claim 1, wherein the starting material is a compound of the formula V in which $T_1$ to $T_4$ are hydrogen.

4. A process according to claim 1, wherein the starting materials are cyanomethylene compounds of the formulae VIII and IX containing the radicals $R_1$ and $R_2$ of the formulae II to IV, in which $X_1$ is hydrogen, bromine, chlorine or carbamoyl, or phenylcarbamoyl unsubstituted or substituted by chlorine or methyl, $X_2$ and $X_3$ are hydrogen, fluorine, bromine or chlorine, or together form a carbodiimino group, wherein an imino group can be substituted by methyl, and $Y_1$, $Y_2$, $Z_1$ and $Z_2$ are hydrogen, bromine, chlorine, methyl or methoxy, and V is imino.

5. A process according to claim 1, wherein the starting materials are cyanomethylene compounds of the formulae VIII and IX containing the radicals $R_1$ and $R_2$ of the formula II, in which $X_1$ to $X_3$ are hydrogen or chlorine.

6. A process according to claim 1, wherein the starting materials are unsubstituted phthalonitrile and cyanomethylene compounds of the formulae

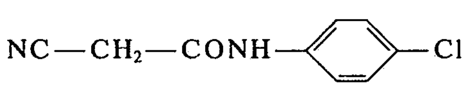

and

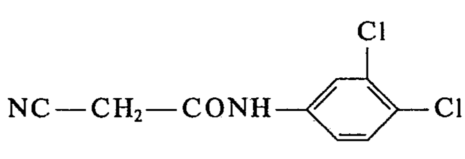

7. A process according to claim 1, wherein the polar solvent is an alcohol.

8. A process according to claim 1, wherein the strong base is an alkali hydroxide, alkali carbonate or alkali alcoholate.

9. A process according to claim 1, wherein the base is used together with a phase-transfer catalyst.

10. A process according to claim 1, wherein the condensation is performed, through all reaction stages, in the same reaction medium.

11. A process according to claim 1, wherein an organic acid is used for the hydrolysis reaction.

**Revendications**

1. Procédé de préparation d'iso-indolines bis-méthiniques de formule I

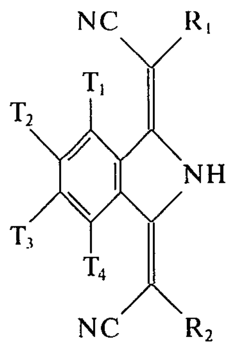

(I)

dans laquelle les symboles $T_1$ à $T_4$ représentent l'hydrogène ou un halogène ou bien l'un ou deux des radicaux $T_1$ et $T_4$ représentent des groupes alkyle, alcoxy ou phénoxy, et $R_1$ et $R_2$ désignent chacun, indépendamment l'un de l'autre, un groupe cyano, alkyl- ou aryl-carbonyloxy, carbamoyle, alkylcarbamoyle ou un radical de formules II à IV ci-dessous:

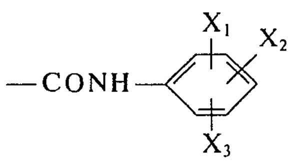
(II)
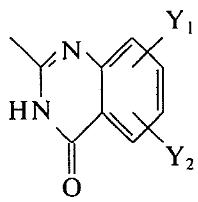
(III)
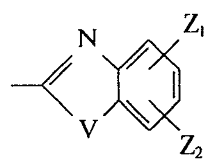
(IV)

(dans lesquelles $X_1$ est un groupe non solubilisant, $X_2$ et $X_3$ désignent chacun l'hydrogène, un halogène, un alkyle, un alcoxy ou un alcoxycarbonyle ou bien forment ensemble un groupe $-NR'-CO-NR''-$, R' et R'' étant, indépendamment l'un de l'autre, l'hydrogène ou un alkyle en $C_1$ à $C_4$, $Y_1$ et $Y_2$ sont chacun l'hydrogène, un halogène, un alkyle ou un alcoxy ou bien forment ensemble un cycle benzénique condensé, V représente l'oxygène, le soufre, un groupe imino ou alkylimino en $C_1$ à $C_4$, et $Z_1$ et $Z_2$ ont les mêmes significations que $Y_1$ et $Y_2$), procédé caractérisé en ce que l'on effectue la condensation de 1 mole d'un composé de formules V, VI ou VII ou d'un sel d'un composé de formule VI ou VII

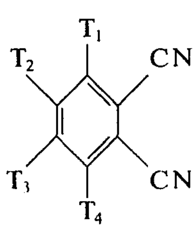
(V)
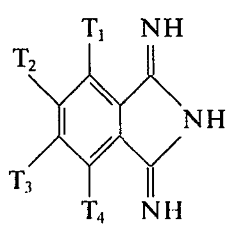
(VI)
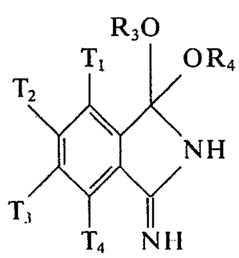
(VII)

(les symboles $T_1$ à $T_4$ ayant les significations ci-dessus et $R_3$ et $R_4$, qui peuvent être identiques ou différents l'un de l'autre, étant des alkyles en $C_1$–$C_4$ ou formant ensemble le groupe $-CH_2-CH_2-$) avec 2 moles d'un composé à groupe cyanométhylène de formule

$NC-CH_2-R_1$ (VIII) ou $NC-CH_2-R_2$ (IX)

($R_1$ et $R_2$ ayant les significations précédemment données) ou avec 1 mole d'un composé cyanométhylène de formule VIII et 1 mole d'un composé cyanométhylène de formule IX dans un solvant polaire en présence d'une base forte, et on isole par hydrolyse l'iso-indoline bis-méthinique formée.

2. Procédé selon la revendication 1, caractérisé en ce que l'on part d'un composé de formules V, VI ou VII dans lequel $T_1$ à $T_4$ sont l'hydrogène et $R_3$ et $R_4$ des groupes méthyle.

3. Procédé selon la revendication 1, caractérisé en ce que l'on part d'un composé de formule V dans

lequel $T_1$ et $T_4$ sont l'hydrogène.

4. Procédé selon la revendication 1, caractérisé en ce que l'on part de composés cyano-méthyléniques de formules VIII et IX dans lesquels $R_1$ et $R_2$ sont des radicaux de formules II à IV, $X_1$ étant l'hydrogène, le brome, le chlore, un groupe carbamoyle ou un groupe phénylcarbamoyle sans substituants ou substitué par le chlore ou un méthyle, $X_2$ et $X_3$ étant l'hydrogène, le fluor, le brome ou le chlore ou bien formant ensemble un groupe carbodiimino dont un radical imino peut être substitué par un méthyle, $Y_1$, $Y_2$, $Z_1$ et $Z_2$ étant l'hydrogène, le brome, le chlore, un méthyle ou un méthoxy, et V un groupe imino.

5. Procédé selon la revendication 1, caractérisé en ce que l'on part de composés à groupes cyanométhylènes de formules VIII et IX dans lesquels $R_1$ et $R_2$ sont des radicaux de formule II, les symboles $X_1$ à $X_3$ représentant l'hydrogène ou le chlore.

6. Procédé selon la revendication 1, caractérisé en ce que l'on part de phthalonitrile sans substituants et de composés cyanométhylèniques de formules

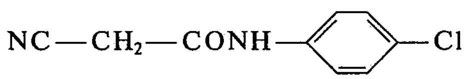

et

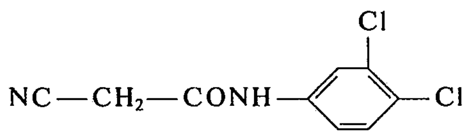

7. Procédé selon la revendication 1, caractérisé en ce que l'on utilise un alcool comme solvant polaire.

8. Procédé selon la revendication 1, caractérisé en ce que l'on utilise comme base forte un hydroxyde, un carbonate ou un alcoolate de métal alcalin.

9. Procédé selon la revendication 1, caractérisé en ce que l'on utilise la base conjointement avec un catalyseur de transfert de phases.

10. Procédé selon la revendication 1, caractérisé en ce que l'on effectue la condensation dans le même milieu pour toutes les étapes réactionnelles.

11. Procédé selon la revendication 1, caractérisé en ce que l'on utilise un acide organique pour l'hydrolyse.